# EUROPEAN PATENT APPLICATION

(11) **EP 4 616 811 A1**
(43) Date of publication of application: **17.09.2025**
(21) Application number: 25162666.9
(22) Date of filing: 10.03.2025
(51) Int. Cl.: A61B 17/06

(54) **PASSING NEEDLES FOR ELASTOMERIC DEVICES**

(30) Priority: 14.03.2024 US 202463565159 P
(71) Applicant: International Life Sciences, LLC d/b/a Artelon, Marietta, Georgia 30067 (US)
(72) Inventor: CAIN, David Brett, Marietta, 30067 (US); FERRARIO, Giovanni Eugenio, Marietta, 30067 (US); SLUDER, Justin C., Marietta, 30067 (US); COLE, Jantzen, Marietta, 30067 (US)
(74) Representative: V.O.

(57) **Abstract**

An orthopedic system and surgical procedure using the orthopedic system. The orthopedic system includes an elastomeric device (e.g. a flat strip of at least one elastomer material) and a passing needle. The passing needle has an opening (e.g. a slot) that is sized and shaped to receive an end of the elastomeric device, with the elastomeric device secured in the opening.

## Description

### RELATED FIELDS

Orthopedic systems and surgical procedures using an elastomeric device secured to a passing needle.

### BACKGROUND

Passing needles for passing suture through soft tissue are known. It is desirable to keep the cross sectional geometry of the passing needle as small as possible to minimize trauma to the soft tissue during use. Typically, the passing needle has been secured to the suture by inserting an end of the suture into a longitudinal cavity of the needle and crimping the needle onto the suture. While this technique works well in some applications, it has drawbacks for other applications. For instance, crimping does not necessarily work well for securing other types of flexible members, such as elastic members and/or flat members. Additionally, crimping does not allow for intra-operative reattachment of the suture or other flexible member after it has been cut or otherwise separated from the passing needle. There remains room for improvement.

### SUMMARY

In one example an orthopedic system includes an elastomeric device and a passing needle. The elastomeric device is a flat strip of at least one elastomer material. The passing needle includes a distal tip, a proximal head, and an elongated shaft extending between the distal tip and the elongated head. The proximal head includes an opening that is sized and shaped to receive an end of the elastomeric device, with the elastomeric device secured to the passing needle while the elastomeric device is located in the opening.

In another example a surgical procedure uses an orthopedic system including an elastomeric device and a passing needle. The elastomeric device is a flat strip of at least one elastomer material. The passing needle includes an opening in a proximal head that is sized and shaped to receive an end of the elastomeric device, with the elastomeric device secured to the passing needle. The surgical procedure includes piercing a soft tissue with a distal tip of the passing needle; passing the passing needle through the soft tissue; and using the passing needle, pulling the elastomeric device at least partially through the soft tissue. The procedure may include, after pulling the elastomeric device at least partially through the soft tissue, separating the elastomeric device from the passing needle. The procedure may include, after separating the elastomeric device from the passing needle, and during the surgical procedure, re-securing the elastomeric device to the same or a different passing needle.

In another example a method for preparing an orthopedic system, such as prior to a surgical procedure, includes providing an elastomeric device and a passing needle. The elastomeric device includes a flat strip of at least one elastomer material. The passing needle includes a distal tip, a proximal head, and an elongated shaft extending between the distal tip and the elongated head, wherein the proximal head comprises an opening in the proximal head that is sized and shaped to receive an end of the elastomeric device. The method includes securing, or re-securing, the elastomeric device to the passing needle with the end of the elastomeric device located in the opening. The passing needle to which the elastomeric device is (re-)secured to may comprise a slot in a proximal head and at least one transverse opening in the proximal head extending through the proximal head and through the slot transverse to the slot. Securing, or re-securing, the elastomeric device may comprise inserting a portion of the elastomeric device into the slot and passing a flexible member, such as a suture, through the transverse opening such that the flexible member pierces the elastomeric device and (re-)secures the elastomeric device to the passing needle.

### BRIEF DESCRIPTION OF FIGURES

FIG. 1 shows an example of an orthopedic system including an elastomeric device connected to two passing needles.
FIG. 2 shows an example of a passing needle.
FIGS. 3 and 4 show another example of a passing needle.
FIGS. 5-7 show the passing needle of FIGS. 3 and 4 in proportion to an elastomeric device.

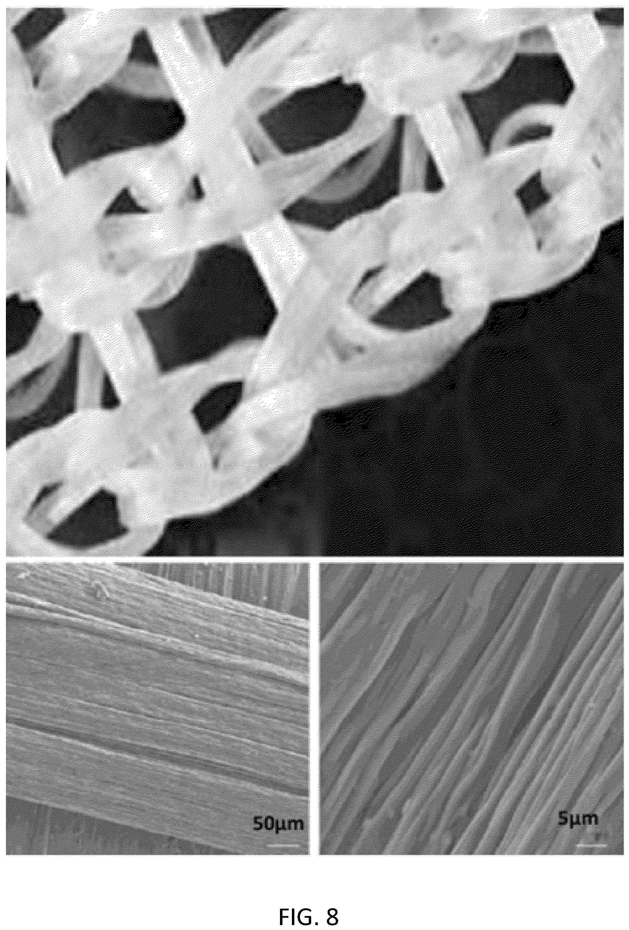

FIG. 8 shows an example of an elastomeric device at different magnifications.

### DETAILED DESCRIPTION

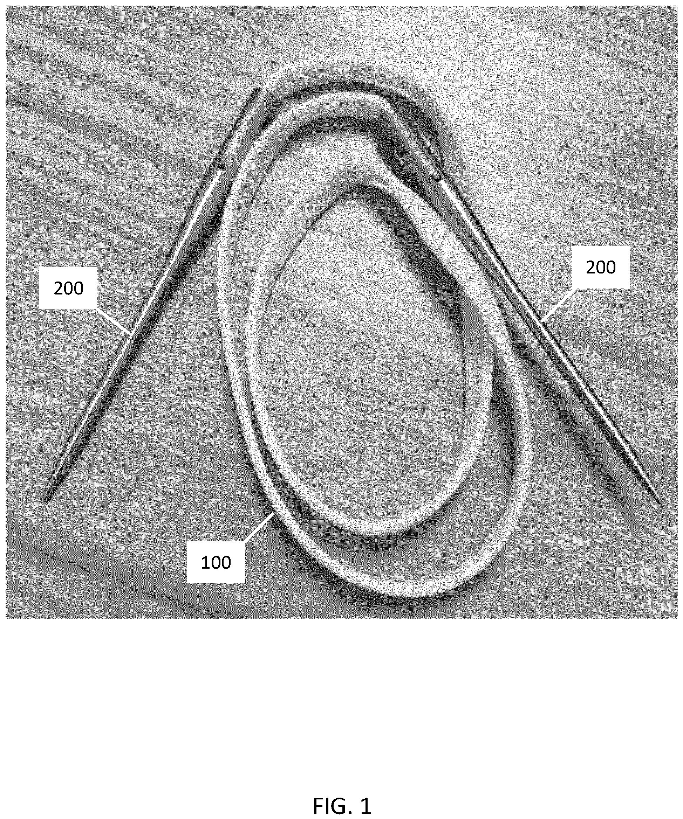

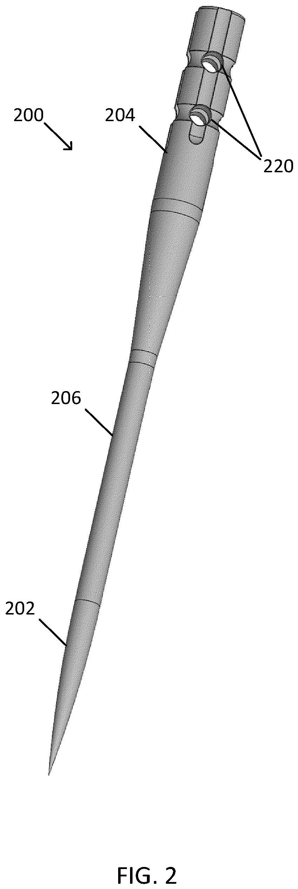

FIG. 1 shows one example of an orthopedic system including an elastomeric device 100 and two passing needles 200. In this example, the elastomeric device 100 is a flat strip of at least one elastomer material, with each of the passing needles 200 secured to one of the ends of the elastomeric device 100. FIG. 2 shows one of the passing needles 200 of FIG. 1 in more detail.

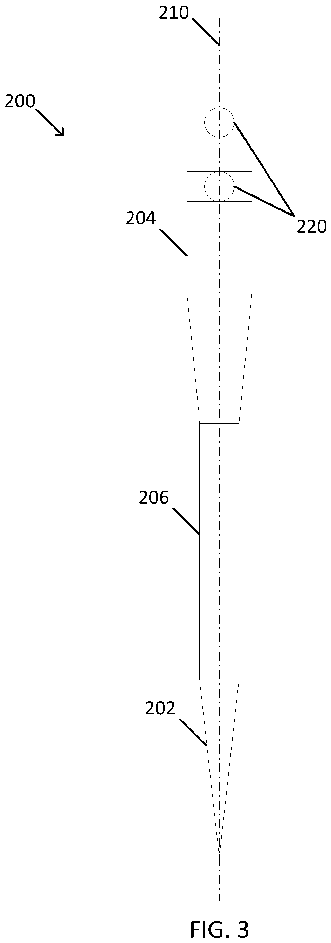

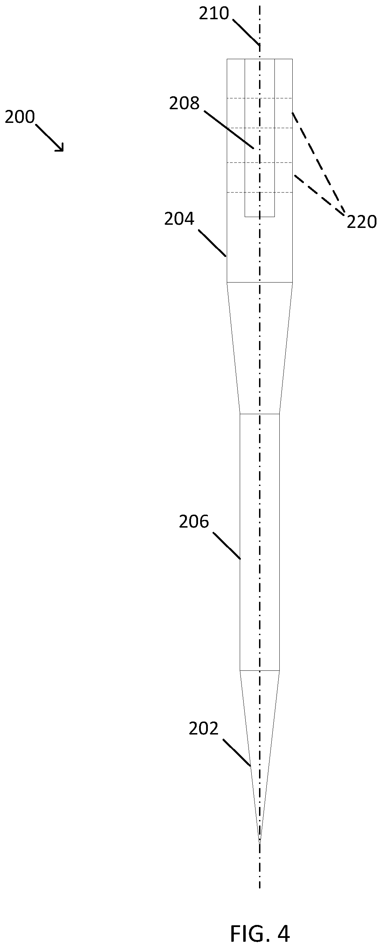

FIGS. 3 and 4 show another example of a passing needle 200. The passing needle 200 has a distal tip 202, a proximal head 204, and an elongated shaft 206 extending between the distal tip 202 and the proximal head 204. The passing needle 200 extends along a longitudinal axis 210. As shown in FIG. 4, the proximal head 204 includes an opening 208 (in this example a slot) that is sized and shaped to receive an end of the elastomeric device 100. The elastomeric device 100 may be secured to the passing needle 100, e.g. prior to a surgical procedure, with an end of the elastomeric device 100 located in the opening 208.

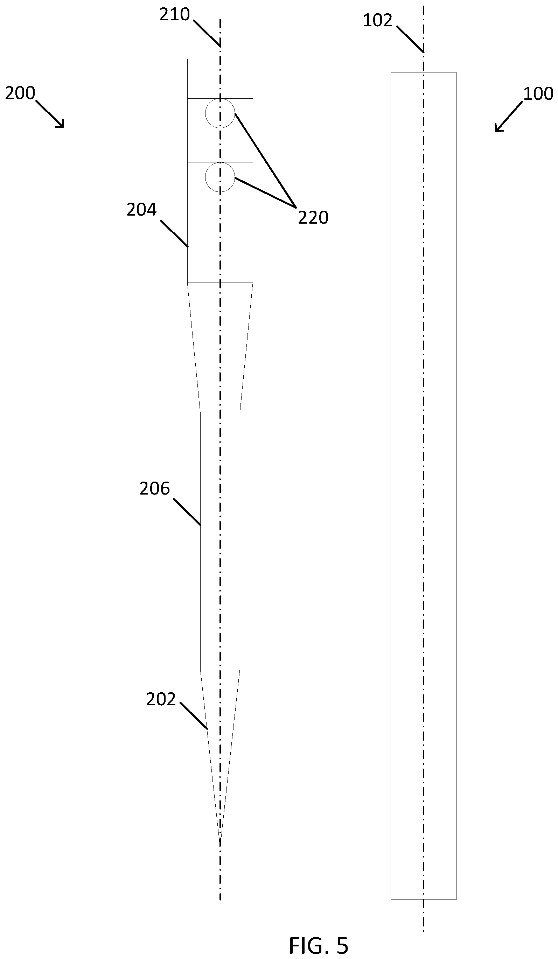

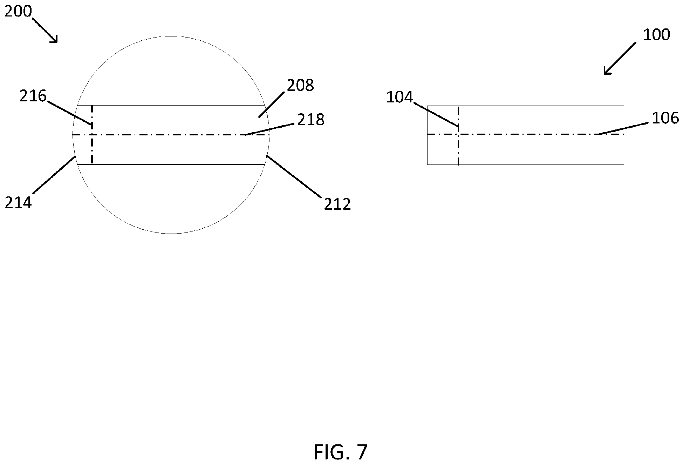

FIGS. 5-7 show the passing needle 200 in relation to and proportion to the elastomeric device 100 that can be secured to the passing needle 200. The opening 208 in the proximal head 204 is sized and shaped to receive the end of the elastomeric device 100 in a flat condition, with the elastomeric device 100 secured to the passing needle 200 with the end of the elastomeric device 100 located in the opening 208 in a flat condition. In this example, the cross sectional shape of the opening 208 in the proximal head 204 of the passing needle 200 is rectangular (with the cross section being perpendicular to the longitudinal axis 210 of the passing needle 200). In this example, the cross sectional shape of the elastomeric device 100 is also rectangular (with the cross section being perpendicular to the longitudinal axis 102 of the elastomeric device 100). FIG. 7 shows the cross sectional shapes of the opening 208 and the elastomeric device 100. As shown in FIG. 7 the opening 208 extends across a width of the proximal head 204 between a first open side 212 and a second open side 214 of the opening 208.

As shown in FIG. 7 the cross sectional shapes and sizes of the opening 208 and the elastomeric device 100 are substantially the same when viewed perpendicular to their longitudinal axes 210 and 102. More particularly, both the opening 208 and the elastomeric device 100 are rectangular in cross section, with the height 216 of the opening 208 being substantially the same as the thickness 104 of the elastomeric device 100, and the width 218 of the opening 208 being substantially the same as the width 106 of the elastomeric device 100. As used herein, "substantially the same" means that there is less than 10% difference between the cross section dimensions of the elastomeric device 100 and the opening 208. In some implementations, having an opening 208 and elastomeric device 100 with substantially the same cross sections facilitates connection of the elastomeric device 100 to the passing needle 200 without undue distortion of the elastomeric device 100, and also facilitates minimization of the cross sectional area of the passing needle's 200 proximal head 204. Although FIG. 7 shows the proximal head 204 having a circular cross section, the cross section area could be further minimized by shortening the dimension of the proximal head 204 transverse to the opening 208. For instance, the cross section of the proximal head 204 could be made more oval or rectangular than shown in FIG. 7.

In the examples shown in the figures, the passing needle 200 also includes transverse openings 220 extending through the proximal head 204 and through the opening 208 in a direction transverse (specifically perpendicular) to the opening 208. The transverse openings 220 are configured to receive one or more flexible members (e.g. sutures) that extend through the transverse openings 200 and pierce the portion of the elastomeric device 100 positioned in the opening 208 to secure the elastomeric device 100 to the passing needle 200.

In some implementations the passing needle 200 described above provides for improved securement of an elastomeric device to a passing needle. For example, earlier techniques such as crimping may not work with elastomeric devices due to their elastic properties (e.g. the elastomeric properties may result in the elastomeric device being easily pulled out of the crimp) whereas the passing needle 200 described above provides effective securement of an elastomeric device 100 despite its elastic properties. Additionally, crimping is a "one time" process, which means that a crimped passing needle cannot be reattached to a suture or other flexible member once it has been removed. The passing needle 200 described above can be reattached intraoperatively if needed, for example by re-suturing the passing needle 200 to the same or different elastomeric device 200 after having been separated from the elastomeric device 200.

### Elastomeric Device

In some implementations, the elastomeric device may be an Artelon^{®} FlexBand.^{®} The elastomeric device may be an elongated, flexible, elastic strip of material. The elastomeric device may be a degradable biomaterial matrix woven from wet-spun fibers of polycaprolactone based-polyurethane urea (PUUR) that have been knitted into textile strips for optimal mechanical properties and ease of use as reinforcement for numerous orthopaedic soft tissue reconstructive applications. The clinical efficacy of the elastomeric device may be generated from the combination of the chemical composition, fiber spinning, and the textile manufacturing process. The PUUR multiblock based copolymer of biomaterial matrix may leverage the well-established biocompatibility of polyurethane biomaterials with the ability to specifically calibrate matrix biodegradation kinetics post-implantation.

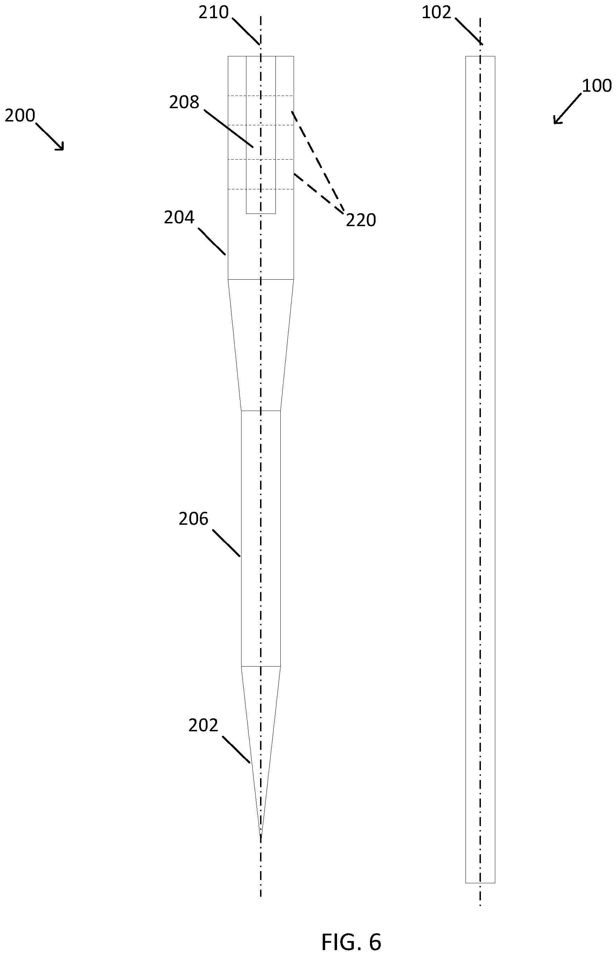

In one example of use, the elastomeric device may be used as a reinforcement device for soft tissue repair where weaknesses exist during tendon or ligament reconstructive procedures. The device's woven matrix may act as a porous tissue scaffold to promote soft tissue support while also encouraging healing. FIG. 6 shows magnified views of one example of such an elastomeric device, showing the knitted fibers and pores of the matrix. In some embodiments, the matrix has a porosity range of approximately 8um-600um, with the majority of the pores concentrated in the smaller subrange, and a texture capable of accommodating matrix producing cells to form a functional tissue.

Historically, soft tissue augmentation devices were designed to have a strong rigid structure and thus, the mechanical properties were not properly matched to the musculoskeletal tissues targeted for reconstruction. This high stiffness profile transfers most of the mechanical load to the augmentation device, which often results in clinical failure due to stress-shielding or device fatigue. In order to prevent the biological breakdown in healing associated with stress shielding, the elastomeric device may be designed to have an original tensile stiffness measuring at least 50% lower than that of the tissue to be reconstructed. The ranges of elasto-mechanical loading profiles of the augmentation device may approximate human ligaments and tendons and yet be more adaptable to deformation than the native tissue. In some implementations, this ensures matrix continuity even if the healing target tissue is overstretched, in which case the damaged target tissue can persist in the healing process while continuing to be supported by the augmentation device. In addition, in some implementations, the generous elastomeric characteristics enable the graft to resist long-term stress relaxation and creep thus, providing the augmentation device with the ability to template the healing tissue to its desired dimension and ensure ultimate functional kinetics.

In some implementations, in addition to the enhanced graft flexibility, the post-implantation endurance profile may ensure that the graft maintains 90% of its original strength and tensile properties for the first year. This ensures that during the acute phase of healing, when the mechanical properties of the regenerating tissue are compromised, the augmentation device will help share loading of the healing tissue, but the tissue itself is still offered adequate mechanical stimuli to induce cells to secrete paracrine factors by the process of mechanotransduction for further cell recruitment, differentiation, and matrix deposition to generate a functional tissue. Simultaneous with maturation of the new tissue, the augmentation device may be configured to gradually and benignly degrades by hydrolysis.

The selection of the proper graft polymer chemistry (i.e., polyurethane urea) and textile manufacturing method of the augmentation device may, in some implementations, enable the biodegradation rate to be advantageously adapted to the expected healing rate and to coincide with the increasing biomechanical properties of the tissue to be reconstructed. In some embodiments, the favorable high strength, load sharing, and elasticity of the augmentation device combined with the unique ability to custom design the graft's biomechanical and biodegradation properties to specifically align with different anatomical locations/tissue types provides an advantage over other non-degradable synthetic grafts.

Studies analyzing the early in vitro degradation kinetics of Artelon fibers and fiber-based devices demonstrated the original gross mass, stiffness, compressibility, and tensile properties are maintained to a minimum of 90% at one year, and 50% out to three years, again emphasizing the endurance of the matrix through the critical acute healing phase.

## Claims

1. An orthopedic system comprising:
(a) an elastomeric device comprising a flat strip of at least one elastomer material;
(b) a passing needle comprising a distal tip, a proximal head, and an elongated shaft extending between the distal tip and the elongated head, wherein the proximal head comprises an opening in the proximal head that is sized and shaped to receive an end of the elastomeric device;
wherein the elastomeric device is secured to the passing needle with the end of the elastomeric device located in the opening.

2. The orthopedic system of claim 1, wherein the distal tip is a narrowed distal tip and wherein the proximal head is an enlarged proximal head.

3. The orthopedic system of claim 1 or 2, wherein the opening in the proximal head is sized and shaped to receive the end of the elastomeric device in a flat condition; and wherein the elastomeric device is secured to the passing needle with the end of the elastomeric device located in the opening in the flat condition.

4. The orthopedic system of claim 1, 2 or 3, wherein the opening in the proximal head comprises a slot in the proximal head.

5. The orthopedic system of claim 4, wherein the passing needle extends along a longitudinal axis and wherein the slot defines a rectangular cross sectional shape perpendicular to the longitudinal axis.

6. The orthopedic system of any of claims 1-5, wherein the elastomeric device extends along a longitudinal axis and wherein the elastomeric device defines a rectangular cross sectional shape perpendicular to the longitudinal axis.

7. The orthopedic system of claim 4, 5, or 6 as far as dependent from claim 4, wherein the slot extends across a width of the proximal head between a first open side of the slot and a second open side of the slot.

8. The orthopedic system of claim 4 or 5, or claim 6 or 7 as far as dependent from claim 4, wherein the slot comprises a height that is substantially the same as a thickness of the elastomeric device

9. The orthopedic system of claim 4 or 5, or claim 6, 7 or 8 as far as dependent from claim 4, wherein the slot comprises a width that is substantially the same as a width of the elastomeric device.

10. The orthopedic system of any of claims 1-9 wherein the proximal head comprises at least one transverse opening extending through the proximal head and through the slot transverse to the slot.

11. The orthopedic system of claim 10, further comprising at least one flexible member extending through the transverse opening and piercing the elastomeric device to secure the elastomeric device to the passing needle.

12. The orthopedic system of claim 11, wherein the at least one flexible member is a suture.

13. The orthopedic system of any of claims 1-12, configured for separating the elastomeric device from the passing needle, and re-securing the elastomeric device to the same or a different passing needle.

14. A method for preparing an orthopedic system, comprising:
providing an elastomeric device comprising a flat strip of at least one elastomer material;
providing a passing needle comprising a distal tip, a proximal head, and an elongated shaft extending between the distal tip and the elongated head, wherein the proximal head comprises an opening in the proximal head that is sized and shaped to receive an end of the elastomeric device; and
securing the elastomeric device to the passing needle with the end of the elastomeric device located in the opening.

15. The method of claim 14, wherein the passing needle to which the elastomeric device is secured to comprises a slot in a proximal head and at least one transverse opening in the proximal head extending through the proximal head and through the slot transverse to the slot, wherein securing the elastomeric device comprises inserting a portion of the elastomeric device into the slot and passing a flexible member, such as a suture, through the transverse opening such that the flexible member pierces the elastomeric device and secures the elastomeric device to the passing needle.
